# FASCICULE DE BREVET EUROPEEN

(11) **EP 0 965 308 B1**
(45) Date de publication et mention de la délivrance du brevet: **01.08.2007**
(21) Numéro de dépôt: 99810507.6
(22) Date de dépôt: 10.06.1999
(51) Int. Cl.: A61B 17/16

(54) **Alésoir de chirurgie**
Chirurgische Reibahle
Surgical reamer

(30) Priorité: 17.06.1998 CH 130298
(43) Date de publication de la demande: 22.12.1999
(73) Titulaire: PRECIMED S.A., 2534 Orvin (CH)
(72) Inventeur: Lechot, André, 2534 Orvin (CH)
(74) Mandataire: Mötteli-Mantelli, Novella

(56) Documents cités:
- EP-A- 0 648 477
- DE-U- 29 616 633
- US-A- 2 746 722
- US-A- 5 122 134

## Description

La présente invention a pour objet un alésoir de chirurgie constitué d'une tige d'entraînement en rotation, munie d'une tête de coupe présentant à l'arrière, des dents de coupe auxiliaires permettant un retrait aisé de l'alésoir.

Les alésoirs sont utilisés en chirurgie pour préparer dans les os des logements destinés à recevoir des implants. Ces alésoirs présentent généralement une tête de coupe conique, plus précisément tronconique, creuse. Cette tête est souvent fixée à l'extrémité d'une tige rigide ou flexible formée par au moins une bande d'acier enroulée en hélice. Le travail de coupe dans la paroi osseuse a pour effet de former des copeaux mélangés à une masse spongieuse. Lors du retrait de l'alésoir par le chirurgien, cette masse a tendance à créer un bourrage qui freine l'alésoir continuant de tourner en exerçant sur celui-ci un couple résistant important. Ce couple résistant peut aller jusqu'à entraîner la rupture d'une partie de l'alésoir. Dans le cas d'une tige flexible, ce couple résistant peut provoquer rapidement une déformation de la tige flexible. La résistance est particulièrement grande lorsque le chirurgien retire l'alésoir en le faisant tourner dans le sens opposé au sens prescrit.

Afin de faciliter le retrait de l'alésoir il a été proposé de former sur une partie tronconique, à l'arrière de la tête de l'alésoir, des dents de coupe auxiliaires. De tels alésoirs sont décrits dans le brevet US 5,122,134. Les dents de coupe auxiliaires de ces alésoirs sont taillées selon le prolongement des hélices des dents principales. Ces dents de coupe auxiliaires permettant un mouvement de retrait aisé de l'alésoir, grâce à l'effet de coupe des dents situées à l'arrière de l'alésoir. Lors du retrait de l'alésoir, les dents auxiliaires coupent les copeaux. La masse spongieuse et les copeaux peuvent s'écouler, sans bourrer, dans les goujures formées entre les dents auxiliaires.

Pour obtenir l'effet favorable visé l'alésoir doit tourner dans un sens déterminé lors de son retrait. Or il arrive fréquemment que le chirurgien retire l'alésoir en le faisant tourner dans le sens opposé au sens prescrit.

La présente invention a pour but de mettre à la disposition du chirurgien un alésoir facile à retirer quelque soit le sens de rotation de telle sorte que le chirurgien n'a pas besoin de se préoccuper du sens de rotation de l'alésoir.

L'alésoir selon l'invention est caractérisé en ce que la coupe des dents auxiliaires est alternativement orientée à gauche et à droite de manière à assurer une coupe dans les deux sens de rotation.

Le dessin annexé représente, à titre d'exemple, un mode d'exécution de l'alésoir selon l'invention.

La figure 1 représente l'alésoir dans une première position angulaire de sa tête de coupe.

La figure 2 représente l'arrière de la tête de coupe dans une seconde position angulaire décalée d'une dent relativement à la première position.

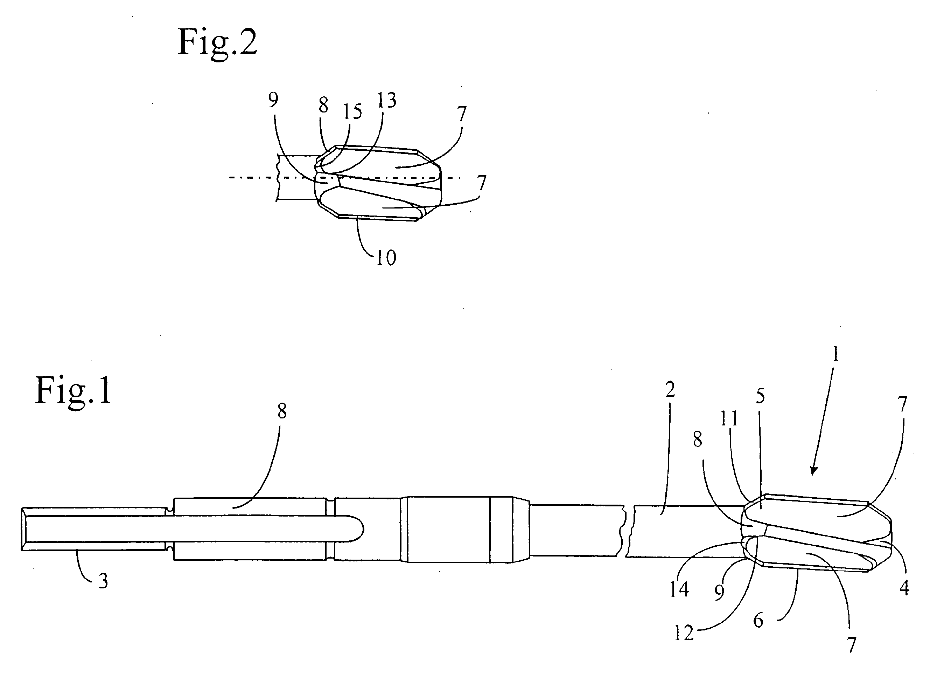

L'alésoir représenté à la figure 1 comprend une tête de coupe 1 formée à l'extrémité d'une tige rigide 2 elle-même soudée à un emmanchement 3 destiné à être relié à une machine d'entraînement. La tête de coupe 1 présente deux extrémités tronconiques 4 et 5 reliées par une partie médiane cylindrique 6. La tête 1 est entièrement traversée par quatre goujures hélicoïdales 7. Les quatre dents de la denture avant 4 ou denture principale sont taillées de manière à couper pour une rotation à droite. La partie tronconique arrière 5 est munie d'une denture taillée de telle de manière que les angles d'attaque et de dépouille des quatre dents sont alternativement d'un côté et de l'autre des dents de telle sorte que cette partie arrière coupe aussi bien dans un sens de rotation que dans l'autre. Sur les figures on voit que les dents 8 et 10 sont taillées de manière à couper lors d'une rotation à droite tandis que les dents voisines 9 et 11 sont taillées de manière à couper lors d'une rotation à gauche. Les arêtes de coupe sont donc, par exemple, respectivement situées sur les côtés 12 et 13 des dents 8 et 9 de même que les dépouilles 14 et 15.

Le retrait de l'alésoir peut se faire soit par une rotation à droite, soit par une rotation à gauche, soit par une rotation droite gauche alternée.

La tige rigide 2 pourrait bien entendu être remplacée par une tige flexible formée de préférence par l'enroulement en hélice, en sens opposés, de deux bandes en acier.

## Revendications

1. Alésoir de chirurgie constitué d'une tige (2) d'entraînement en rotation, munie d'une tête de coupe (1) présentant, à l'arrière, des dents de coupe auxiliaires (5) permettant un retrait aisé de l'alésoir, **caractérisé en ce que** la coupe des dents auxiliaires (5) est alternativement orientée à gauche et à droite de manière à assurer une coupe dans les deux sens de rotation.

## Claims

1. Asurgical reamer consisting of a rod (2) driven in rotation, equipped with a cutting head (1) having, toward the rear, auxiliary cutting teeth (5) permitting easy withdrawal of the reamer, wherein the cutting edges of the auxiliary teeth (5) are oriented both leftward and rightward in such a way as to ensure cutting in both directions of rotation.

## Patentansprüche

1. Chirurgische Reibahle bestehend aus einem Drehschaft (2) mit einem Schneidkopf (1), der an seiner Rückseite Hilfsschneidezähne (5) aufweist, mit denen die Reibahle leicht herausgezogen werden kann, **dadurch gekennzeichnet, dass** die Hilfszähne (5), um ein Schneiden in beide Drehrichtungen sicherzustellen, abwechselnd links und rechts geschliffen sind.
